(19) Europäisches Patentamt
European Patent Office
Office européen des brevets

(11) Veröffentlichungsnummer: **0 117 481**
**A1**

(12) # EUROPÄISCHE PATENTANMELDUNG

(21) Anmeldenummer: **84101525.8**

(22) Anmeldetag: **15.02.84**

(51) Int. Cl.³: **C 07 D 207/456,** A 01 N 43/36

(30) Priorität: **26.02.83 DE 3306844**

(43) Veröffentlichungstag der Anmeldung: **05.09.84**
**Patentblatt 84/36**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Marzolph, Gerhard, Dr., Semmelweisstrasse 87A, D-5000 Köln 80 (DE)**
Erfinder: **Blank, Heinz Ulrich, Dr., Am Geusfelde 35, D-5068 Odenthal (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen 1 (DE)**
Erfinder: **Hänssler, Gerd, Dr., Heymannstrasse 40, D-5090 Leverkusen 1 (DE)**

(54) **Fungizide Mittel auf Maleinsäureimid-Basis.**

(57) Die Erfindung betrifft die fungizide Verwendung der teilweise bekannten Maleinsäureimid-Derivate der Formel (I)

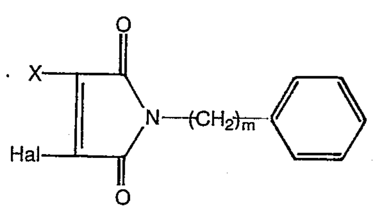

(I)

in welcher
X für Wasserstoff oder Halogen,
Hal für Halogen und
m für eine ganze Zahl von 2 bis 4 stehen.

EP 0 117 481 A1

ACTORUM AG

BAYER AKTIENGESELLSCHAFT 5090 Leverkusen, Bayerwerk

Konzernverwaltung RP
Patentabteilung Bas/bc/c

II

# Fungizide Mittel auf Maleinsäureimid-Basis

Die Erfindung betrifft die Verwendung der teilweise bekannten Maleinsäureimid-Derivate als Fungizide.

Es ist bereits bekannt, daß halogensubstituierte N-Aralkyl-maleinsäureimide, wie das 2,3-Dichlormaleinsäure-N-(2-phenylethyl)-imid, als antibakteriell- und antiparasitär-wirksame Verbindungen auf dem Pharmasektor eingesetzt werden (vgl. US-Patent 3 129 225). Weiterhin ist das 2,3-Dichlormaleinsäure-N-(3-phenyl-propyl)-imid als Einsatzstoff in der Fotochemie bekannt (vgl. N. Boens, J. Polym. Sci., Polym. Chem. Ed. 1975, 13 (1), 201-203 (Engl.)).

Das Maleinsäure-N-(2-phenylethyl)-imid ist als Saatgutbeizmittel bekannt (vgl. Jap. Patent 78-27336).

Es wurde nun gefunden, daß die teilweise bekannten Maleinsäure-N-aralkyl-imide der Formel (I)

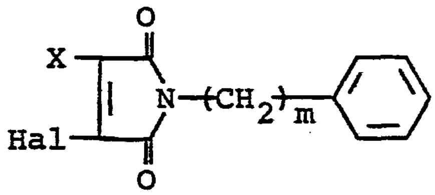

(I)

in welcher
X für Wasserstoff oder Halogen,
Hal für Halogen und
m für eine ganze Zahl von 2 bis 4 stehen,

gute fungizide Wirkungen auf dem Pflanzenschutzgebiet aufweisen.

Überraschenderweise zeigen die Maleinsäure-N-aralkylimide der Formel (I) eine breitere und bessere Wirkung gegen Pilze als die aus dem Stand der Technik bekannten Verbindungen.

Die neue Verwendung der Wirkstoffe stellt somit eine Bereicherung der Technik dar.

Die erfindungsgemäß verwendbaren Verbindungen sind durch die Formel (I) definiert. In der Formel stehen vorzugsweise
X für Wasserstoff, Fluor, Chlor oder Brom,
Hal für Fluor, Chlor oder Brom und
m für die Zahl 2, 3 oder 4.

Besonders bevorzugt sind die Verbindungen der Formel (I), in der
X für Wasserstoff, Chlor oder Brom,

Hal für Chlor oder Brom und
m für 2, 3 oder 4 stehen.

Einige der erfindungsgemäßen Verbindungen sind neu, sie können jedoch nach prinzipiell bekannten Verfahren hergestellt werden, z.B. durch Umsetzen

a) eines Halogenmaleinsäureanhydrids der Formel (II)

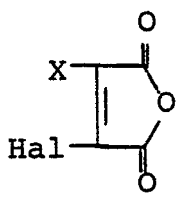

(II)

in welcher
X und Hal die oben angegebene Bedeutung haben,

mit primären Aminen der Formel (III)

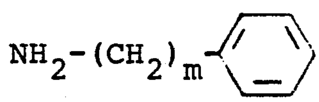

(III)

in welcher
m die oben angegebene Bedeutung hat,

in einem Verdünnungsmittel, oder

b) eines Halogenmaleinsäuredialkylesters der Formel (IV)

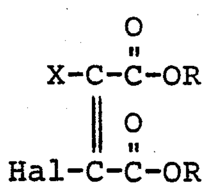

(IV)

in welcher
X und Hal die oben angegebene Bedeutung haben, und
R für Alkyl steht,

mit primären Aminen der Formel (III), in welcher

m die oben angegebene Bedeutung hat,

gegebenenfalls in einem Lösungs- oder Verdünnungsmittel, oder

c) indem man Halogenmaleinsäuremonoamide der Formel (V)

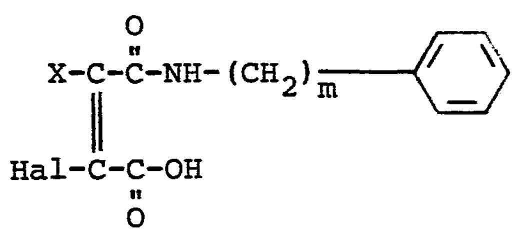

(V)

in welcher
X, Hal und m die oben angegebene Bedeutung haben,

in Gegenwart eines Lösungsmittels, wie z.B. Eisessig und gegebenenfalls eines anhydridisierenden Mittels, wie z.B. Acetanhydrid oder Thionylchlorid zu den Verbindungen der Formel (I) cyclisiert.

Die bei der Durchführung der Verfahrensvariante a) als Ausgangsverbindungen benötigten Halogenmaleinsäureanhydride sind durch die Formel (II) allgemein definiert. Diese Verbindungen sind im Handel erhältlich und/oder nach bekannten Verfahren leicht herstellbar. Die weiterhin bei den Verfahrensvarianten a) und b) einzusetzenden Amine sind durch die Formel (III) definiert. Die Amine sind teilweise bekannt oder können nach allgemein bekannten Verfahren hergestellt werden. So können die Amine beispielsweise durch Reduktion von Nitrilen oder Aldoximen mit Wasserstoff hergestellt werden (vgl. Houben-Weyl, Methoden der organischen Chemie, XI/1, Seiten 341 ff). Weiterhin ist eine gängige Methode die reduktive Aminierung von Aldehyden mit Wasserstoff und Ammoniak (vgl. Houben-Weyl, XI/1, Seite 341). Zur Herstellung von Aralkylaminen eignet sich außerdem die Methode der Reduktion von ω-Nitrostyrolen (vgl. Can. J. Chem. 51 (1973) Seite 1402).

Die bei der Verfahrensvariante b) noch benötigten Halogenmaleinsäuredialkylester sind durch die Formel (IV) beschrieben. Diese Ester sind bekannt und nach gängigen Verfahren aus den käuflichen Halogenmaleinsäureanhydriden durch Umsetzung mit Alkoholen erhältlich.

Die bei der Verfahrensvariante c) einzusetzenden Halogenmaleinsäuremonoamide der Formel (V) sind teilweise bekannt oder können nach an sich bekannten Verfahren aus den entsprechenden Maleinsäureanhydriden durch Umsetzung mit primären Aminen hergestellt werden (vgl. Organic Synthesis 41, Seite 93 (1961)).

Als Verdünnungsmittel kommen für die Verfahrensvariante a) vor allem Carbonsäuren in Frage, wie z.B. Ameisensäure, Essigsäure und Propionsäure.

Als Verdünnungsmittel kommen bei der Verfahrensvariante b) organische Lösungsmittel in Frage. Hierzu gehören vorzugsweise Toluol, Xylol, Chlorbenzol, Perchlorethan, Dioxan, Glykoldimethylether, Dimethylformamid.

Bei der Verfahrensvariante c) werden vorzugsweise als Verdünnungsmittel eingesetzt: Carbonsäuren, wie Essigsäure; aromatische Kohlenwasserstoffe, wie Toluol, Xylol; Halogenkohlenwasserstoffe, wie Chlorbenzol; weiterhin Dioxan und als anhydridisierende Reagenzien vorzugsweise Acetanhydrid, Phosgen, Thionylchlorid, Phosphoroxichlorid, Phosphorpentachlorid.

Die Reaktionstemperaturen können bei der Durchführung der verschiedenen Verfahrensvarianten in einem größeren Bereich variiert werden. Bei Verfahrensvariante a) arbeitet man bei Temperaturen von 20 bis 150°C, vorzugsweise von 80 bis 120°C. Bei Verfahrensvariante b) arbeitet man bei Temperaturen von 50 bis 180°C, vorzugsweise von 80 bis 130°C und bei der Verfahrensvariante c) bei Temperaturen von 0 bis 150°C, vorzugsweise von 50 bis 120°C.

Bei allen drei Varianten wird im allgemeinen bei Normaldruck gearbeitet.

Bei der Durchführung aller Verfahrensvarianten werden die Ausgangsstoffe vorzugsweise in äquimolaren Mengen eingesetzt.

Nach einer bevorzugten Ausführungsform der Verfahrensvariante a) werden die Ausgangsstoffe in äquimolaren Mengen in einem organischen Lösungsmittel, z.B. Eisessig, mehrere Stunden bei erhöhter Temperatur gerührt. Anschließend auf Raumtemperatur abgekühlt, Wasser wird zugesetzt, wobei das Produkt bereits ausgeschieden wird.

Setzt man das Dibrommaleinsäureanhydrid als Ausgangsstoff ein, so wird dieses in einer bevorzugten Ausführungsform in der Lösung aus Dibrommaleinsäure in Eisessig unter Rühren hergestellt und in dieser Lösung direkt weiter mit dem Amin umgesetzt.

Nach einer bevorzugten Ausführungsform der Verfahrensvariante b) wird der Dihalogenmaleinsäuredialkylester aus Dihalogenmaleinsäureanhydrid und Methanol hergestellt und dieser nach fraktionierter Destillation mit dem Amin umgesetzt. Die Aufarbeitung erfolgt wie oben beschrieben (vgl. US-Patent 3 734 927, Beispiel 5).

Die erfindungsgemäß verwendbaren Wirkstoffe weisen eine starke mikrobizide Wirkung auf und können zur Bekämpfung von unerwünschten Mikroorganismen praktisch eingesetzt werden. Die einzusetzenden Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes,

Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Venturia-Arten z.B. gegen den Erreger des Apfelschorfs (Venturia inaequalis), zur Bekämpfung von Leptosphaeria-Arten, wie z.B. den Erreger der Braunfleckenkrankheit des Weizens (Leptosphaeria nodorum), zur Bekämpfung von Oomyceten, wie z.B. den Erreger der Kartoffel- und Tomatenkrautfäule (Phytophthora infestans), zur Bekämpfung von Reiskrankheiten, wie z.B. Pyricularia oryzae, zur Bekämpfung von Puccinia-Arten, z.B. gegen den Erreger des Weizenbraunrostes (Puccinia recondita), eingesetzt werden.
Weiterhin ist die fungizide Wirkung gegen Cochliobolus sativus und Pyrenophora teres an Getreide sowie die Wirkung gegen Erreger von Keimlingskrankheiten (wie z.B. Rhizoctonia solani) bei verschiedenen Kulturpflanzen wie z.B. Baumwolle, Leguminosen u.a. zu nennen. Bei entsprechenden Konzentrationen sind die Verbindungen auch akarizid wirksam.
Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe,

Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkoholether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Anwendungsbeispiele

In den nachfolgenden Beispielen werden die nachstehend angegebenen Verbindungen als Vergleichssubstanzen eingesetzt:

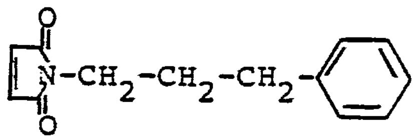
(A)

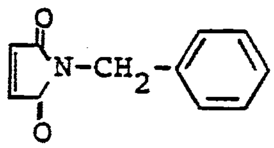
(B)

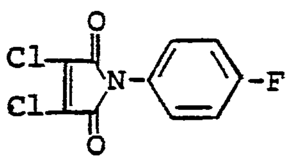
(C)

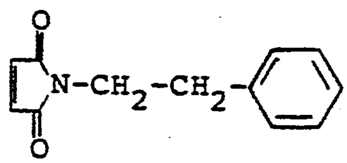
(D)

Beispiel A

Puccinia-Test (Weizen) / protektiv

Lösungsmittel: 100 Gewichtsteile Dimethylformamid
Emulgator: 0,25 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer rel. Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß der Herstellungsbeispiele : 2, 4, 5, 1, 3 .

Tabelle A

Puccinia-Test (Weizen) / protektiv

| Wirkstoff | Wirkstoffkonzentration in der Spritzbrühe in Gew.-% | Krankheitsbefall in % der unbehandelten Kontrolle |
|---|---|---|
| 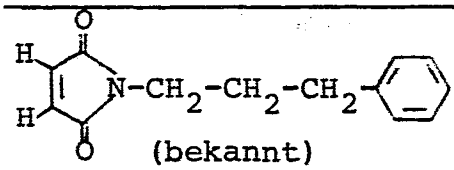 (A) (bekannt) | 0,025 | 58,7 |
| 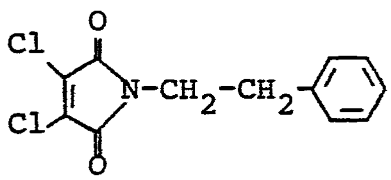 (2) | 0,025 | 0,0 |
| 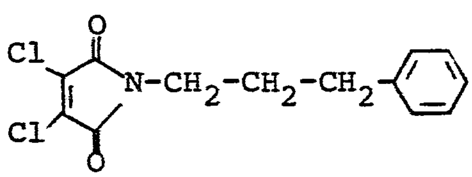 (4) | 0,025 | 21,3 |
| 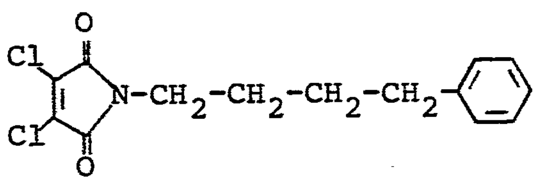 (5) | 0,025 | 25,0 |
| 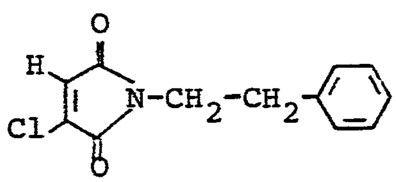 (1) | 0,025 | 25,0 |
| 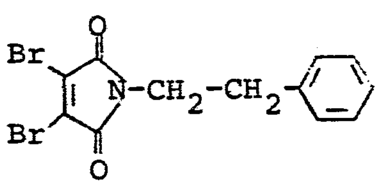 (3) | 0,025 | 25,0 |

Beispiel B

Leptosphaeria nodorum-Test (Weizen) / protektiv

Lösungsmittel: 100 Gewichtsteile Dimethylformamid
Emulgator: 0,25 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß der Herstellungsbeispiele: 2, 4, 1, 3..

Tabelle B

Leptosphaeria nodorum-Test (Weizen) / protektiv

| Wirkstoff | Wirkstoffkonzentration in der Spritzbrühe in Gew.-% | Krankheitsbefall in % der unbehandelten Kontrolle |
|---|---|---|
| 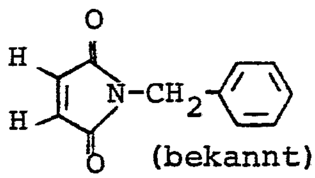 (B) (bekannt) | 0,025 | 100 |
| 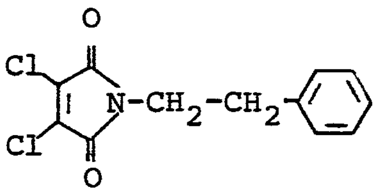 (2) | 0,025 | 0,0 |
| 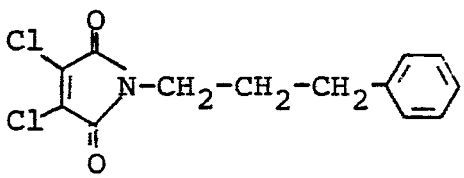 (4) | 0,025 | 20,0 |
| 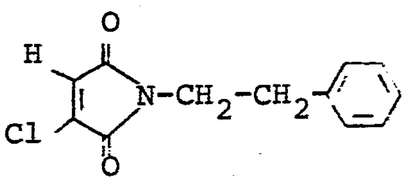 (1) | 0,025 | 16,7 |
| 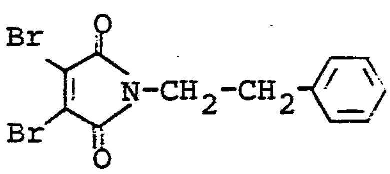 (3) | 0,025 | 25,0 |

Beispiel C

Phytophthora-Test (Tomate) / protektiv

Lösungsmittel: 4,7 Gewichtsteile Aceton
Emulgator: 0,3 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % rel. Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele: 2 und 5.

Tabelle C

Phytophthora-Test (Tomate) / protektiv
systemisch

| Wirkstoff | Befall in % bei einer Wirkstoffkonzentration von 250 ppm |
|---|---|
| 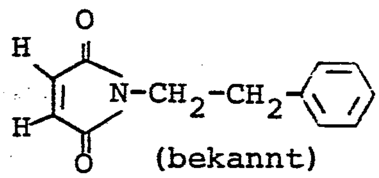 (D) (bekannt) | 100 |
| 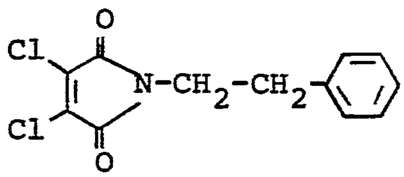 | 12 |
| 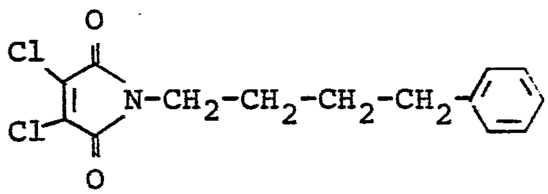 | 50 |

Beispiel D

Venturia-Test (Apfel) / protektiv

Lösungsmittel: 4,7 Gewichtsteile Aceton
Emulgator: 0,3 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele: 2 und 3.

Tabelle D

Venturia-Test (Apfel) / protektiv

| Wirkstoff | Befall in % bei einer Wirkstoffkonzentration von 25 ppm |
|---|---|
| 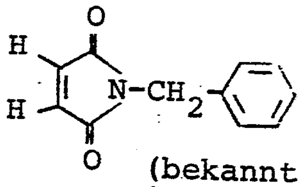 (B) (bekannt) | 60 |
| 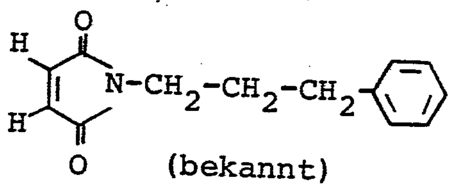 (A) (bekannt) | 68 |
| 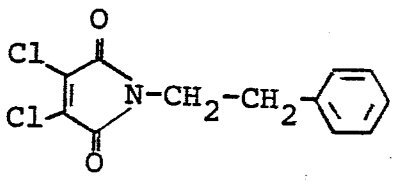 | 37 |
| 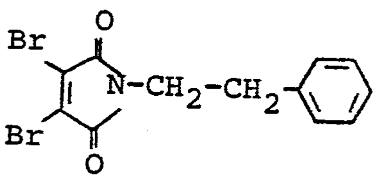 | 10 |

Beispiel E

Pyricularia-Test (Reis) / protektiv

Lösungsmittel: 12,5 Gewichtsteile Aceton
Emulgator: 0,3 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % relativer Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß Herstellungsbeispiel 1.

Tabelle E

Pyricularia-Test (Reis) / protektiv

| Wirkstoffe | | Wirkstoffkonzentration in % | Krankheitsbefall in % der unbehandelten Kontrolle |
|---|---|---|---|
| 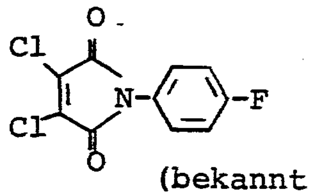 (bekannt) | (C) | 0,025 | 100 |
| 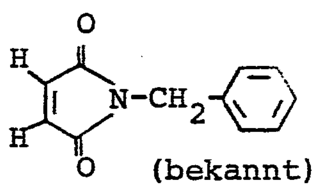 (bekannt) | (B) | 0,025 | 100 |
| 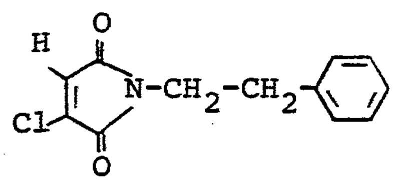 | (1) | 0,025 | 20 |

## Herstellungsbeispiel

### Beispiel 1

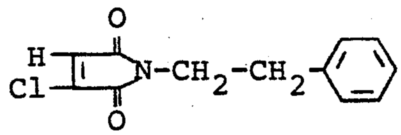

26,5 g (0,2 Mol) Chlormaleinsäureanhydrid wird in 100 ml Eisessig gelöst. Man tropft anschließend 24,2 g (0,2 Mol) 1-Amino-2-phenyl-ethan zu und rührt 4 Stunden bei 100°C nach, kühlt und verrührt die Reaktionsmischung mit 200 ml Wasser. Der Niederschlag wird abgesaugt und aus 150 ml Ethanol umkristallisiert. Man erhält 26,4 g Chlormaleinsäure-N-(2-phenylethyl)-imid (56 % der Theorie) mit dem Schmelzpunkt 119°C.

Analog können die folgenden Verbindungen der Formel (I)

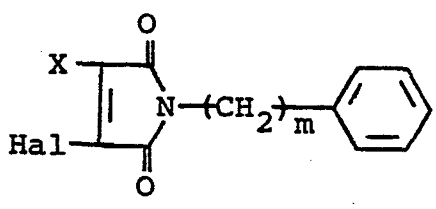

(I)

hergestellt werden:

| Beispiel Nr. | X | Hal | m | Schmelzpunkt (°C) |
|---|---|---|---|---|
| 2 | Cl | Cl | 2 | 131-134 |
| 3 | Br | Br | 2 | 168-169 |
| 4 | Cl | Cl | 3 | 50- 51 |
| 5 | Cl | Cl | 4 | 70- 71 |

Patentansprüche

1) Fungizide Mittel, gekennzeichnet durch einen Gehalt an mindestens einem Maleinsäure-N-aralkyl-imid der Formel (I)

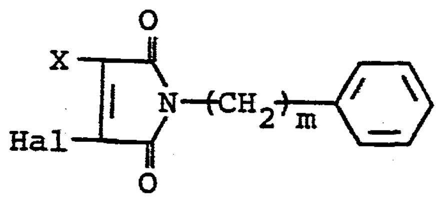

(I)

in welcher
X für Wasserstoff oder Halogen,
Hal für Halogen und
m für eine ganze Zahl von 2 bis 4 stehen.

2) Fungizide Mittel, gekennzeichnet durch einen Gehalt an mindestens einem Maleinsäure-N-aralkyl-imid der Formel (I) gemäß Anspruch 1, in welcher
X für Wasserstoff, Fluor, Chlor oder Brom,
Hal für Fluor, Chlor oder Brom und
m für eine ganze Zahl von 2 bis 4 stehen.

3) Fungizide Mittel, gekennzeichnet durch einen Gehalt an mindestens einem Maleinsäure-N-aralkyl-imid der Formel (I) gemäß Anspruch 1, in welcher
X für Wasserstoff, Chlor oder Brom,
Hal für Chlor oder Brom und
m für eine ganze Zahl von 2 bis 4 stehen.

4) Verfahren zur Bekämpfung von Pilzen, dadurch gekennzeichnet, daß man Maleinsäure-N-aralkyl-imide

der Formel (I) gemäß Ansprüchen 1 bis 3 auf Pilze oder ihren Lebensraum einwirken läßt.

5) Verwendung von Maleinsäure-N-aralkyl-imiden der Formel (I) gemäß Ansprüchen 1 bis 3 zur Bekämpfung von Pilzen.

6) Verfahren zur Herstellung von fungiziden Mitteln zur Bekämpfung von Pilzen, dadurch gekennzeichnet, daß man Maleinsäure-N-aralkyl-imide der Formel (I) gemäß Ansprüchen 1 bis 3 mit Streckmitteln und/ oder oberflächenaktiven Mitteln vermischt.

7) Maleinsäure-N-aralkyl-imid der Formel (I), in welcher X für Wasserstoff, Hal für Chlor und m für die Zahl 2 stehen.

8) Maleinsäure-N-aralkyl-imid der Formel (I), in welcher X und Hal für Brom und m für die Zahl 2 stehen.

9) Maleinsäure-N-aralkyl-imid der Formel (I), in welcher X und Hal für Chlor und m für Zahl 4 stehen.

Europäisches Patentamt

# EUROPÄISCHER RECHERCHENBERICHT

0117481

Nummer der Anmeldung

EP 84 10 1525

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | GB-A- 880 555 (ICI)<br>* Seite 4, "provisional specification" * | 1-9 | C 07 D 207/456<br>A 01 N 43/36 |
| X | GB-A-2 087 879 (SHOWA DENKO K.K.)<br>* Seite 1, "specification" * | 1-9 | |
| X | EP-A-0 045 907 (BAYER AG)<br>* Seiten 1,2 * | 1-9 | |
| D,X | US-A-3 129 225 (S.L. SHAPIRO)<br>* Spalte 1; Spalte 4, Tafel 1 * | 1-9 | |
| P,X | EP-A-0 098 953 (BAYER AG)<br>* Insgesamt * | 1-9 | RECHERCHIERTE SACHGEBIETE (Int. Cl. 3)<br>C 07 D 207/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 04-03-1984 | MAISONNEUVE J.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82